# EUROPEAN PATENT APPLICATION

(11) **EP 3 466 415 A1**
(43) Date of publication of application: **10.04.2019**
(21) Application number: 16903857.7
(22) Date of filing: 14.12.2016
(51) Int. Cl.: A61K 31/167, A61K 35/00, C08G 69/48

(54) **POLYMER CA4 BONDING PHARMACEUTICAL COMPOUND AND PREPARATION METHOD THEREFOR**

(30) Priority: 31.05.2016 CN 201610379051
(71) Applicant: Changchun Institute Of Applied Chemistry Chinese Academy Of Sciences, Changchun, Jilin 130022 (CN)
(72) Inventor: TANG, Zhaohui, Changchun City Jilin 130022 (CN); SONG, Wantong, Changchun City Jilin 130022 (CN); YU, Haiyang, Changchun City Jilin 130022 (CN); NIU, Yuewei, Changchun City Jilin 130022 (CN); ZHANG, Dawei, Changchun City Jilin 130022 (CN); MA, Sheng, Changchun City Jilin 130022 (CN); ZHANG, Yu, Changchun City Jilin 130022 (CN); CHEN, Xuesi, Changchun City Jilin 130022 (CN)
(74) Representative: Witte, Weller & Partner Patentanwälte mbB
(86) International application number: PCT/CN2016/109812
(87) International publication number: WO 2017/206477

(57) **Abstract**

This invention provides a polymer-bonded CA4 pharmaceutical compound and a preparation method therefor. The polymer-bonded CA4 drug provided in this invention has a structure represented by formula (I). With respect to the bonded pharmaceutical compound provided by this invention, CA4 is grafted onto a specific polymer carrier, so that the resultant bonded drug may be enriched in tumor vessels and the active drug is slowly released. Therefore, an efficacy of destroying tumor vessels is exerted at a tumor site for a long period, an excellent tumor inhibition effect is achieved, and the problem of poor therapeutic effects due to short action time of CA4P is effectively solved, and thus it has a broad prospect of development in the field of tumor treatment. Additionally, the preparation method provided in this invention is simple and has wide sources of raw materials, and it is possible to achieve scale production and industrialization.

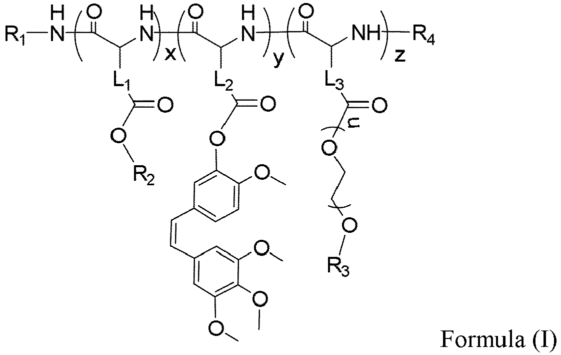

## Description

### TECHNICAL FIELD

This invention relates to the field of pharmaceutical synthesis, and particularly to a polymer-bonded CA4 pharmaceutical compound and a preparation method therefor.

### BACKGROUND ART

Combretastatin ((Z)-3,4,5,4'-tetramethoxy-3'-hydroxydiphenylethylene, Combretastatin A4, CA4) is a novel antitumorally active compound developed recently, and its structural formula is as follows:

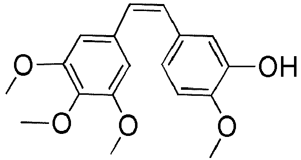

Unlike conventional cytotoxin-type anticancer drugs, CA4 does not directly kill tumor cells. CA4 utilizes a new anticancer mechanism, wherein blood vessels within the tumor are destroyed, the supply of blood and nutrients to the tumor is blocked, and severe necrosis within the tumor is induced, by binding to tubulins in tumor vascular endothelial cells. Due to the structural difference between tumor vessels and normal tissue vessels, CA4 selectively destroys tumor vessels and substantially has no effect on blood supply of normal tissues. Accordingly, high expectation is given by the anticancer field on this type of drugs.

Since the water solubility of CA4 is poor, it is difficult to be directly intravenously administered, Pettit et al. designed and synthesized a phosphated disodium salt precursor drug of CA4, i.e. Combretastatin A4 phosphate disodium (CA4P), in 1995, of which the structure is as follows:

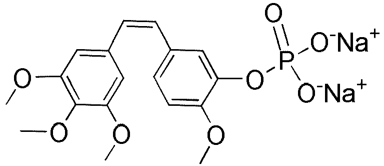

CA4P has greatly improved the water solubility and pharmacokinetic properties of CA4. By using the characteristic that a phosphatase has a concentration in proliferated vascular endothelial cells higher than that in normal cells, CA4P is selectively activated in tumor vessels and CA4 is targetedly released to exert anti-angiogenic and anti-tumor effects. In a series of mouse tumor models, systemic administration of CA4P can rapidly and selectively block tumor vessels, and can usually obtain better therapeutic effects in combination with a conventional chemotherapy, radiation therapy, thermotherapy, and the like. At present, the patent owner of CA4P, OXiGENE Corporation, United States, has finished Phase II clinical trials of CA4P, and has entered Phase III clinical trials with respect to thyroid cancer. However, there are still problems, such as high blood clearance rate, short action time, recurrence after drug withdrawal, and the like, in clinical use of CA4P. It has been found in studies that CA4 has a reversible microtubule inhibition effect, the change of vascular endothelial cells caused thereby can be rapidly recovered after drugs being removed, and small molecules CA4P and CA4 have relatively short residence time in tissues. Tumor, which is different from normal tissues, has excessive growth and a large amount of expression of angiogenesis factors, so that the speed of angiogenesis on the surface of tumor is very high. Therefore, long-term and effective tumor vessel inhibition is required, otherwise the tumor may still rapidly grow. Rapid clearance *in vivo* and insufficient tumor site residence of CA4P both severely impact the long-term effect of tumor vessel inhibition exerted thereby. Accordingly, the problem to be currently solved is to obtain an anti-tumor drug, which is a CA4-type vascular disrupting agent capable of having a long action time at a tumor site.

### SUMMARY OF THE INVENTION

In view of this, the technical problem to be solved by this invention is to provide a polymer-bonded CA4 drug and a preparation method therefor. The polymer-bonded CA4 drug provided by this invention may reside and accumulate in a tumor site for a long time.

This invention provides a polymer-bonded CA4 pharmaceutical compound, having a structure represented by formula (I), 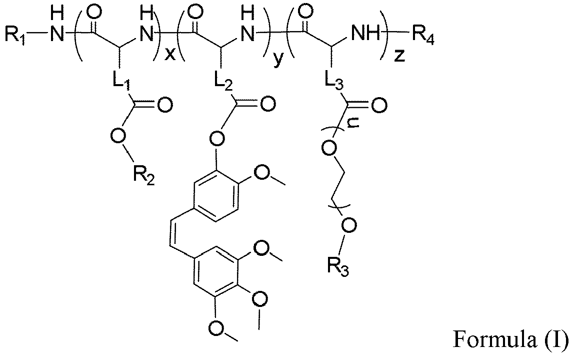 wherein,
R₁ is selected from a C2-C10 linear alkyl group, a C3-C10 branched alkyl group, or a C6-C20 aryl group;
R₂ is selected from a hydrogen atom or a cation;
R₃ is selected from an unsubstituted C1-C20 alkyl group or a substituted C1-C20 alkyl group;
R₄ is selected from a hydrogen atom or a C1-C6 alkyloyl group;
L₁, L₂, and L₃ are independently selected from -CH₂- or -CH₂CH₂-;
x, y, and z represent polymerization degree, and 10 ≤ x+y+z ≤ 5000, wherein x ≥ 0, y > 0, z > 0; and
n represents polymerization degree, and 10 ≤ n ≤ 500.

Preferably, said R₁ is a C3-C8 linear alkyl group, a C5-C8 branched alkyl group, or a C8-C15 aryl group.

Preferably, said R₂ is selected from a hydrogen atom, a metal cation, or an organic cation.

Preferably, said R₂ is selected from a hydrogen atom, a sodium ion, a potassium ion, an ammonium ion, or a positively charged amino acid ion.

Preferably, said R₃ is selected from an unsubstituted C2-C20 linear alkyl group, an unsubstituted C3-C20 branched alkyl group, a substituted C2-C20 linear alkyl group, or a substituted C3-C20 branched alkyl group.

Preferably, the substituent in said substituted C2-C20 linear alkyl group is one or more of a hydroxy group, an aldehyde group, an amino group, a mercapto group, and a saccharide residue; and

the substituent in said substituted C3-C20 branched alkyl group is one or more of a hydroxy group, an aldehyde group, an amino group, a mercapto group, and a saccharide residue.

Preferably, said R₄ is selected from a hydrogen atom, an acetyl group, or a propionyl group.

Preferably, a value range of said x, y, and z is 30 ≤ x+y+z ≤ 300.

This invention further provides a preparation method of a polymer-bonded CA4 pharmaceutical compound, comprising:
reacting a copolymer compound having a structure of formula (II) with CA4 in the presence of a condensation agent 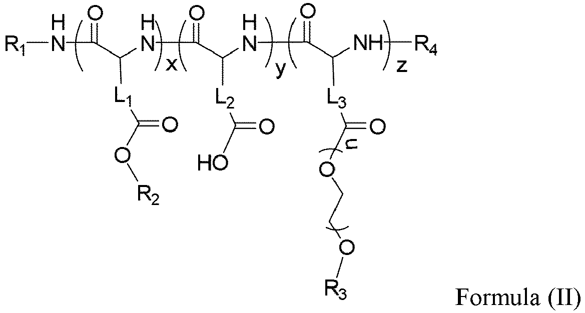 to obtain a polymer-bonded CA4 pharmaceutical compound having a structure of formula (I): 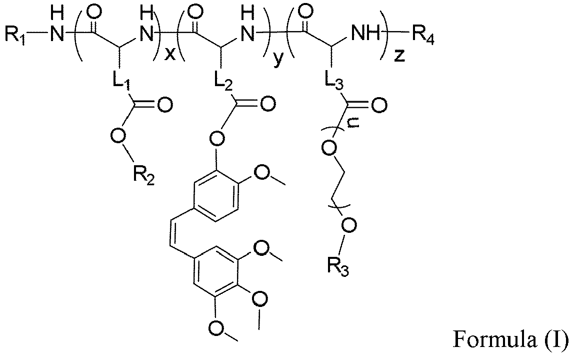 wherein,
R₁ is selected from a C2-C10 linear alkyl group, a C3-C10 branched alkyl group, or a C6-C20 aryl group;
R₂ is selected from a hydrogen atom or a cation;
R₃ is selected from an unsubstituted C1-C20 alkyl group or a substituted C1-C20 alkyl group;
R₄ is selected from a hydrogen atom or a C1-C6 alkyloyl group;
L₁, L₂, and L₃ are independently selected from -CH₂- or -CH₂CH₂-;
x, y, and z represent polymerization degree, and 10 ≤ x+y+z ≤ 5000, wherein x ≥ 0, y > 0, z > 0; and
n represents polymerization degree, and 10 ≤ n ≤ 500.

Preferably, said condensation agent is 2,4,6-trichlorobenzoyl chloride, N,N-diisopropyl carbodiimide, or dicyclohexyl carbodiimide.

Compared to the prior art, the polymer-bonded CA4 pharmaceutical compound provided by this invention has a structure represented by formula (I). With respect to the bonded pharmaceutical compound provided by this invention, CA4 is grafted onto a specific polymer carrier, so that the resultant bonded drug may be enriched in tumor vessels and the active drug is slowly released. Therefore, an efficacy of destroying tumor vessels is exerted at a tumor site for a long period, an excellent tumor inhibition effect is achieved, and the problem of not good enough therapeutic effect due to short action time of CA4P is effectively solved, which has a broad prospect of development in the field of tumor treatment. Furthermore, the preparation method provided by this invention is simple and has wide sources of raw materials, and it is possible to achieve scale production and industrialization.

### DESCRIPTION OF DRAWINGS

Fig. 1 shows ¹HNMR of poly L-glutamic acid grafted with polyethylene glycol prepared in Example 3.
Fig. 2 shows ¹HNMR of poly(glutamic acid) grafting with polyethylene glycol-CA4 bonded pharmaceutical compound prepared in Example 10.
Fig. 3 shows HPLC graphs of a small molecule CA4 and a polymer-bonded CA4 pharmaceutical compound prepared in Example 10.
Fig. 4 shows a dynamic light scattering result of a polymer-bonded CA4 pharmaceutical compound prepared in Example 10 at a concentration of 0.2 mg/mL in water.
Fig. 5 shows a release result of a bonded pharmaceutical compound prepared in Example 10 in a simulated body fluid.
Fig. 6 shows CA4 drug concentrations in tumor tissues after administration of a polymer-bonded CA4 pharmaceutical compound and CA4P in Example 23.
Fig. 7 shows a tumor pathological analysis after single administration of CA4P and a polymer-bonded CA4 pharmaceutical compound measured in Example 24, and
Fig. 8 shows the therapeutic effect of a polymer-bonded CA4 pharmaceutical compound and CA4P on tumor measured in Example 25.

### DESCRIPTION OF EMBODIMENTS

This invention provides a polymer-bonded CA4 pharmaceutical compound having a structure represented by formula (I), 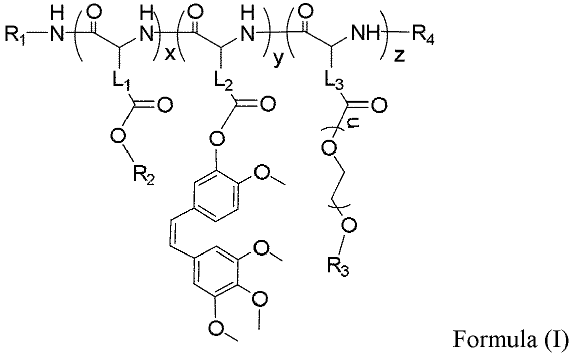 wherein,
R₁ is selected from a C2-C10 linear alkyl group, a C3-C10 branched alkyl group, or a C6-C20 aryl group;
R₂ is selected from a hydrogen atom or a cation;
R₃ is selected from an unsubstituted C1-C20 alkyl group or a substituted C1-C20 alkyl group;
R₄ is selected from a hydrogen atom or a C1-C6 alkyloyl group;
L₁, L₂, and L₃ are independently selected from -CH₂- or -CH₂CH₂-;
x, y, and z represent polymerization degree, and 10 ≤ x+y+z ≤ 5000, wherein x ≥ 0, y > 0, z > 0; and
n represents polymerization degree, and 10 ≤ n ≤ 500.

According to this invention, R₁ is preferably a C3-C8 linear alkyl group, a C5-C8 branched alkyl group, or a C8-C15 aryl group, and more preferably an ethyl group, an *n*-propyl group, an isopropyl group, an *n*-butyl group, an isobutyl group, a *t*-butyl group, an *n*-pentyl group, an isopentyl group, an *n*-hexyl group, an *n*-heptyl group, an *n*-octyl group, a phenyl group, a naphthyl group, a biphenyl group, or an anthracenyl group.

R₂ is preferably a hydrogen atom, a metal cation, or an organic cation, and more preferably a hydrogen atom, a sodium ion, a potassium ion, an ammonium ion, or a positively charged amino acid ion.

R₃ is preferably an unsubstituted C2-C20 linear alkyl group, an unsubstituted C3-C20 branched alkyl group, a substituted C2-C20 linear alkyl group, or a substituted C3-C20 branched alkyl group, and more preferably an unsubstituted C4-C10 linear alkyl group, an unsubstituted C5-C10 branched alkyl group, a substituted C4-C10 linear alkyl group, or a substituted C5-C10 branched alkyl group, wherein the substituent in the substituted C2-C20 linear alkyl group is one or more of a hydroxy group, an aldehyde group, an amino group, a mercapto group, and a saccharide residue; and the substituent in the substituted C3-C20 branched alkyl group is one or more of a hydroxy group, an aldehyde group, an amino group, a mercapto group, and a saccharide residue. More particularly, R₃ is a methyl group, an ethyl group, an *n*-propyl group, an isopropyl group, an *n*-butyl group, an isobutyl group, a *t*-butyl group, an *n*-pentyl group, an isopentyl group, an *n*-hexyl group, an *n*-heptyl group, an *n*-octyl group, a hydroxymethyl group, or a hydroxyethyl group.

R₄ is preferably a hydrogen atom, a formyl group, am acetyl group, a propionyl group, or a butanoyl group.

x, y, z, and n represent polymerization degree; wherein y > 0, z > 0, x ≥ 0; preferably, x > 10; y > 20; z > 4; wherein the range of the sum of x, y, and z is preferably 30 ≤ x+y+z ≤ 300, and more preferably 50 ≤ x+y+z ≤ 250, yet further preferably 75 ≤ x+y+z ≤ 200, and most preferably 100 ≤ x+y+z ≤ 150; n is preferably 20 ≤ n ≤ 400, and more preferably 30 ≤ n ≤ 300, yet further preferably 50 ≤ n ≤ 260, and most preferably 80 ≤ n ≤ 180.

This invention further provides a preparation method of a polymer-bonded CA4 pharmaceutical compound, comprising:
reacting a copolymer compound having a structure of formula (II) with CA4 in the presence of a condensation agent, 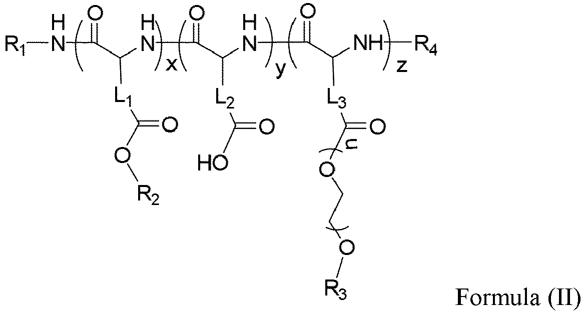 to obtain a polymer-bonded CA4 drug having a structure of formula (I): 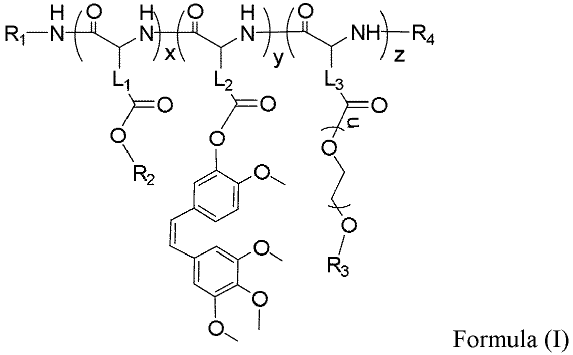 wherein,
R₁ is selected from a C2-C10 linear alkyl group, a C3-C10 branched alkyl group, or a C6-C20 aryl group;
R₂ is selected from a hydrogen atom or a cation;
R₃ is selected from an unsubstituted C1-C20 alkyl group or a substituted C1-C20 alkyl group;
R₄ is selected from a hydrogen atom or a C1-C6 alkyloyl group;
L₁, L₂, and L₃ are independently selected from -CH₂- or -CH₂CH₂-;
x, y, and z represent polymerization degree, and 10 ≤ x+y+z ≤ 5000, wherein x ≥ 0, y > 0, z > 0; and
n represents polymerization degree, and 10 ≤ n ≤ 500.

According to this invention, a copolymer compound having a structure of formula (II) is reacted with CA4 in the presence of a condensation agent in this invention to obtain a polymer-bonded CA4 pharmaceutical compound having a structure of formula (I); wherein the source of the copolymer compound having a structure of formula (II) is not specifically limited in this invention, and it may be prepared by the person skilled in the art according to general knowledge well known to the person skilled in the art. In the copolymer compound having a structure of formula (II), R₁ is preferably a C3-C8 linear alkyl group, a C5-C8 branched alkyl group, or a C8-C15 aryl group, and more preferably an ethyl group, an *n*-propyl group, an isopropyl group, an *n*-butyl group, an isobutyl group, a *t*-butyl group, an *n*-pentyl group, an isopentyl group, an *n*-hexyl group, an *n*-heptyl group, an *n*--octyl group, a phenyl group, a naphthyl group, a biphenyl group, or an anthracenyl group; R₂ is preferably a hydrogen atom, a metal cation, or an organic cation, and more preferably a hydrogen atom, a sodium ion, a potassium ion, an ammonium ion, or a positively charged amino acid ion; R₃ is preferably an unsubstituted C2-C20 linear alkyl group, an unsubstituted C3-C20 branched alkyl group, a substituted C2-C20 linear alkyl group, or a substituted C3-C20 branched alkyl group, and more preferably an unsubstituted C4-C10 linear alkyl group, an unsubstituted C5-C10 branched alkyl group, a substituted C4-C10 linear alkyl group, or a substituted C5-C10 branched alkyl group, wherein the substituent in the substituted C2-C20 linear alkyl group is one or more of a hydroxy group, an aldehyde group, an amino group, a mercapto group, and a saccharide residue; and the substituent in the substituted C3-C20 branched alkyl group is one or more of a hydroxy group, an aldehyde group, an amino group, a mercapto group, and a saccharide residue. More particularly, R₃ is a methyl group, an ethyl group, an *n*-propyl group, an isopropyl group, an *n*-butyl group, an isobutyl group, a *t*-butyl group, an *n*-pentyl group, an isopentyl group, an *n*-hexyl group, an *n*-heptyl group, an *n*-octyl group, a hydroxymethyl group, or a hydroxyethyl group. R₄ is preferably a hydrogen atom, a formyl group, an acetyl group, a propionyl group, or a butanoyl group. X, y, z, and n represent polymerization degree; wherein y > 0, z > 0, x ≥ 0; preferably, x > 10; y > 20; z > 4; wherein the range of the sum of x, y, and z is preferably 30 ≤ x+y+z ≤ 300, and more preferably 50 ≤ x+y+z ≤ 250, yet further preferably 75 ≤ x+y+z ≤ 200, and most preferably 100 ≤ x+y+z ≤ 150; n is preferably 20 ≤ n ≤ 400, and more preferably 30 ≤ n ≤ 300, yet further preferably 50 ≤ n ≤ 260, and most preferably 80 ≤ n ≤ 180. The condensation agent is preferably 2,4,6-trichlorobenzoyl chloride, N,N-diisopropyl carbodiimide, or dicyclohexyl carbodiimide; and a solvent of the reaction is preferably one or more of N,N-dimethylformamide, dimethyl sulfoxide, chloroform, and dichloromethane. The temperature of the reaction is preferably 10-60°C, and more preferably 20-40°C; and the time of the reaction is preferably 2-60 hours, more preferably 10-30 hours.

The polymer-bonded CA4 pharmaceutical compound provided by this invention has a structure represented by formula (I). With respect to the bonded drug provided by this invention, CA4 is grafted onto a specific polymer carrier, so that the resultant bonded drug may be enriched in tumor vessels and the active drug is slowly released. Therefore, an efficacy of destroying tumor vessels is exerted at a tumor site for a long period, an excellent tumor inhibition effect is achieved, and the problem of not good enough therapeutic effect due to short action time of CA4P is effectively solved, which has a broad prospect of development in the field of tumor treatment. Additionally, the preparation method of a pharmaceutical compound provided by this invention is simple and has wide sources of raw materials, and it is possible to achieve scale production and industrialization.

A more clear and complete description will be made below in conjunction with the technical solutions in Examples of this invention. Obviously, the Examples described are merely part of the Examples of this invention, rather than all of the Examples. Based on the Examples in this invention, all other Examples obtained by those of ordinary skill in the art without performing inventive work belong to the scope protected by this invention.

### Example 1

42.1 g (160.0 mmol) of a γ-benzyl-L-glutamate-N-carboxylic anhydride monomer (BLG-NCA) was dissolved in 270mL of anhydrous N,N-dimethylformamide (DMF). 1.0 mL (1.0 mmol/L solution in DMF) of n-hexylamine (n-HA) was added after dissolution with stirring. After sealing, the reaction was performed with stirring for 72h at temperature of 25°C. After completion of the reaction, the resultant reaction solution was taken in 2.0L of ethyl ether, filtered and washed with ethyl ether, and vacuum-dried at room temperature for 24h sequentially to give the intermediate product poly(y-benzyl-L-glutamate) (PBLG).

10.0 g of poly(y-benzyl-L-glutamate) prepared above was dissolved with 100mL of dichloroacetic acid, 30mL of a hydrogen bromide/glacial acetic acid solution with a mass content of 33% was added while stirring, and the reaction was performed with stirring for 1h at temperature of 30°C. Thereafter, the resultant reaction solution was taken in 1.0L of ethyl ether, and centrifuged. The resultant precipitate was re-dissolved with DMF, then dialyzed with deionized water, and freeze-dried to give poly(L-glutamic acid) homopolymer (PLG).

The resultant poly(L-glutamic acid) homopolymer was subjected to nuclear magnetic resonance analysis using deuterated water as a deuterated agent. The results indicated that a chemical shift of 4.43 ppm was a signal peak of a methine group on the main chain, a chemical shift of 2.21 ppm was a signal peak of a methylene group on a side group that was connected to a carbonyl group, and chemical shifts of 1.91 ppm and 1.71 ppm were signal peaks of methylene groups on a side group that were connected to the main chain. As calculated according to the nuclear magnetic resonance spectra, the resultant poly(L-glutamic acid) had a polymerization degree of 135 and an overall yield of 81.2%.

### Example 2

42.1 g (160.0 mmol) of a γ-benzyl-L-glutamate-N-carboxylic anhydride monomer (BLG-NCA) was dissolved in 270mL of anhydrous N,N-dimethylformamide (DMF). 1.0 mL (1.0 mmol/L Solution in DMF) of n-hexylamine (n-HA) was added after dissolution with stirring. After sealing, the reaction was performed with stirring for 72h at temperature of 25°C. Thereafter, 2.0 g (20.0 mmol) of acetic anhydride was added to the above reaction system for continuing the reaction for 6h. After completion of the reaction, the resultant reaction solution was taken in 2.0L of ethyl ether, sequentially filtered and washed with ethyl ether, and vacuum-dried at room temperature for 24h to give the intermediate product poly(y-benzyl-L-glutamate) (PBLG).

10.0 g of poly(y-benzyl-L-glutamate) prepared above was dissolved with 100mL of dichloroacetic acid, 30mL of a hydrogen bromide/glacial acetic acid solution with a mass content of 33% was added while stirring, and the reaction was performed with stirring for 1h at temperature of 30°C. Thereafter, the resultant reaction solution was taken in 1.0L of ethyl ether, and centrifuged. The resultant precipitate was re-dissolved with DMF, then dialyzed with deionized water, and freeze-dried to give acetyl-capped poly(L-glutamic acid) homopolymer (PLG).

### Example 3

Poly(L-glutamic acid) (1.7 g, 13.2 mmol glutamic acid units) prepared in Example 1 and 3.5 g (79.5 mmol ethylene glycol units) of polyethylene glycol monomethyl ether (5000Da) were added into a dry reaction bottle, and dissolved by further adding 150mL of DMF. Thereafter, 178mg (1.4 mmol) of N,N-diisopropyl carbodiimide (DIC) and 196mg (1.6 mmol) of 4-dimethylaminopyridine (DMAP) were added, a sealed reaction was performed at temperature of 25°C, and the resultant reaction solution was taken in 1.0L of ethyl ether after 48 hours. The resultant solid was re-dissolved with DMF, then dialyzed with deionized water for 3 days, and freeze-dried to give poly(L-glutamic acid) grafted with polyethylene glycol having a structure of formula (II).

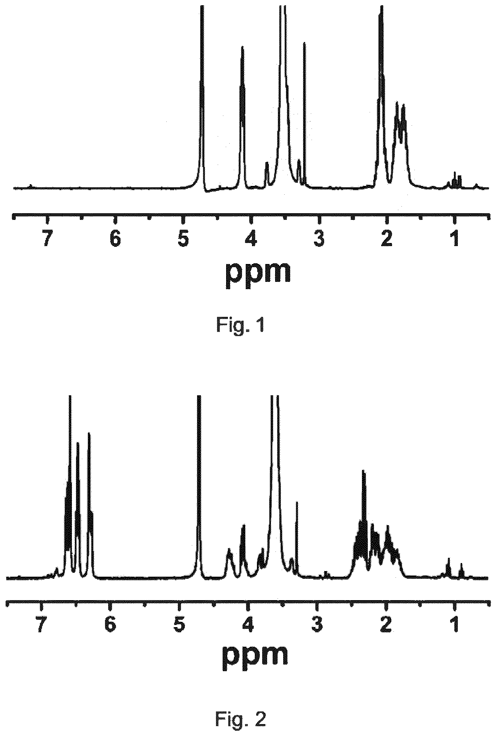

The resultant poly(L-glutamic acid) grafted with polyethylene glycol was subjected to nuclear magnetic resonance analysis by using deuterated water as a solvent, and the results could be seen in Fig. 1. Fig. 1 showed ¹HNMR of poly(L-glutamic acid) grafted with polyethylene glycol prepared in Example 3. As could be seen from the figure, positions of peaks included: δ 4.25 ppm (t, -CH<), 3.63 ppm (t, -CH₂CH₂O-), 3.31 ppm (s, -OCH₃), 2.18 ppm (m, -CH₂COOH), 1.96 and 1.83 ppm (m, > CHCH₂-), 1.10 - 1.02 ppm (m, -CH₂CH₂-), 0.78 ppm (t, -CH₂-CH₃). As could be seen, a raw material for a polyamino acid grafted with polyethylene glycol had a structure of formula (II).

### Example 4

Poly(L-glutamic acid) (1.7 g, 13.2 mmol glutamic acid units) prepared in Example 2 and 3.5 g (79.5 mmol ethylene glycol units) of polyethylene glycol monomethyl ether (2000Da) were added into a dry reaction bottle, and dissolved by further adding 150mL of DMF. Thereafter, 178mg (1.4 mmol) of N,N-diisopropyl carbodiimide (DIC) and 196mg (1.6 mmol) of 4-dimethylaminopyridine (DMAP) were added, a sealed reaction was performed at temperature of 25°C, and the resultant reaction solution was taken in 1.0L of ethyl ether after 48 hours. The resultant solid was re-dissolved with DMF, then dialyzed with deionized water for 3 days, and freeze-dried to give poly(L-glutamic acid) grafted with polyethylene glycol.

### Example 5

24.9 g (100.0 mmol) of a γ-benzyl-L-aspartate-N-carboxylic anhydride monomer (BLA-NCA) was dissolved in 270mL of anhydrous dichloromethane. 1.0 mL (1.0 mmol/L Solution in DMF) of n-hexylamine (n-HA) was added after dissolution with stirring. After sealing, the reaction was performed with stirring for 72h at temperature of 25°C. After completion of the reaction, the resultant reaction solution was taken in 2.0L of ethyl ether, filtered and washed with ethyl ether, and vacuum-dried at room temperature for 24h sequentially to give the intermediate product poly(y-benzyl-L-aspartate) (PBLA).

10.0 g of poly(y-benzyl-L-aspartate) prepared above was dissolved with 100mL of dichloroacetic acid, 30mL of a hydrogen bromide/glacial acetic acid solution with a mass content of 33% was added while stirring, and the reaction was performed with stirring for 1h at temperature of 30°C. Thereafter, the resultant reaction solution was taken in 1.0L of ethyl ether, and centrifuged. The resultant precipitate was re-dissolved with DMF, then dialyzed with deionized water, and freeze-dried to give poly(L-aspartic acid) homopolymer (PLA).

### Example 6

24.9 g (100.0 mmol) of a γ-benzyl-L-aspartate-N-carboxylic anhydride monomer (BLA-NCA) was dissolved in 270mL of anhydrous dichloromethane. 1.0 mL (1.0 mmol/L Solution in DMF) of n-hexylamine (n-HA) was added after dissolution with stirring. After sealing, the reaction was performed with stirring for 72h at temperature of 25°C. Thereafter, 2.0 g of acetic anhydride was added to the above reaction system for continuing the reaction for 6h. After completion of the reaction, the resultant reaction solution was taken in 2.0L of ethyl ether, sequentially filtered and washed with ethyl ether, and vacuum-dried at room temperature for 24h to give the intermediate product poly(y-benzyl-L-aspartate) (PBLA).

10.0 g of poly(y-benzyl-L-aspartate) prepared above was dissolved with 100mL of dichloroacetic acid, 30mL of a hydrogen bromide/glacial acetic acid solution with a mass content of 33% was added while stirring, and the reaction was performed with stirring for 1h at temperature of 30°C. Thereafter, the resultant reaction solution was taken in 1.0L of ethyl ether, and centrifuged. The resultant precipitate was re-dissolved with DMF, then dialyzed with deionized water, and freeze-dried to give acetyl-capped poly(L-aspartic acid) homopolymer (PLA).

### Example 7

1.5 g (13.2 mmol aspartic acid units) of poly(L-aspartic acid) prepared in Example 5 and 3.0 g (68.1 mmol ethylene glycol units) of polyethylene glycol monomethyl ether (10000Da) were added into a dry reaction bottle, and dissolved by further adding 150mL of a mixed solvent of dimethyl sulfoxide/dichloromethane. Thereafter, 178mg (1.4 mmol) of N,N-diisopropyl carbodiimide (DIC) and 196mg (1.6 mmol) of 4-dimethylaminopyridine (DMAP) were added, a sealed reaction was performed at temperature of 25°C, and the resultant reaction solution was taken in 1.0L of ethyl ether after 48 hours. The resultant solid was re-dissolved with DMF, then dialyzed with deionized water for 3 days, and freeze-dried to give poly(L-aspartic acid) grafted with polyethylene glycol.

### Example 8

1.5 g (13.2 mmol aspartic acid units) of poly(L-aspartic acid) prepared in Example 6 and 3.0 g (68.1 mmol ethylene glycol units) of polyethylene glycol monomethyl ether (2000Da) were added into a dry reaction bottle, and dissolved by further adding 150mL of DMF. Thereafter, 178mg (1.4 mmol) of N,N-diisopropyl carbodiimide (DIC) and 196mg (1.6 mmol) of 4-dimethylaminopyridine (DMAP) were added, a sealed reaction was performed at temperature of 25°C, and the resultant reaction solution was taken in 1.0L of ethyl ether after 48 hours. The resultant solid was re-dissolved with DMF, then dialyzed with deionized water for 3 days, and freeze-dried to give poly(L-aspartic acid) grafted with polyethylene glycol.

### Examples 9-11

### Preparation of poly(glutamic acid) grafting with polyethylene glycol-CA4 bonded pharmaceutical compound

Poly(glutamic acid) grafted with polyethylene glycol (585mg) prepared in Example 3 was separately added to three dry reaction bottles, and dissolved with 20ml of dry N,N-dimethylformamide. Thereafter, dry triethylamine (0.153 mL) and 2,4,6-trichlorobenzoyl chloride (0.172 mL) were added. After placing in an oil bath at 60°C, stirring was performed for 10min. Thereafter, 316mg, 253mg, or 190mg of CA4 and 135mg of 4-dimethylaminopyridine were added respectively under a nitrogen atmosphere for continuing the reaction at room temperature for 12h. After completion of the reaction, the reaction solution was taken in ethyl ether, and filtered. The solid was collected and vacuum-dried at room temperature. The solid was re-dissolved with DMF, and freeze-dried to give poly(glutamic acid) grafting with polyethylene glycol-CA4 bonded pharmaceutical compound. The weight was measured, and the yield was calculated.

A resultant polymer-bonded CA4 drug was subjected to nuclear magnetic resonance analysis using deuterated water as a deuterated agent. The results could be seen in Fig. 2. Fig. 2 was a hydrogen nuclear magnetic resonance spectrogram of poly(glutamic acid) grafting with polyethylene glycol-CA4 bonded pharmaceutical compound prepared in Example 10. Compared to Fig. 1, apparent CA4 characteristic peaks (6.29 ppm, 6.44 ppm, 6.60 ppm) could be found, suggesting that CA4 was successfully bonded onto a polymer.

By using ultraviolet-visible light spectrograms, the bonding content of CA4 in bonded pharmaceutical compounds obtained in Examples 9-11 was obtained, the maximal absorption peak of CA4 was at 295nm. The calculation formula for the content of CA4 (%) was: (mass of CA4 bonded pharmaceutical compound/total mass of bonded pharmaceutical compound) x 100%. The results could be seen in Table 1. Table 1 showed the preparation method yield of bonded pharmaceutical compounds provided in Examples 9-11 of this invention and CA4 contents obtained in the bonded pharmaceutical compounds.

**Table 1**

| Example | CA4 content (%) | Yield (%) |
|---|---|---|
| 9 | 34.6 | 85.0 |
| 10 | 28.4 | 86.3 |
| 11 | 21.0 | 88.8 |

### Examples 12-14

### Preparation of poly(glutamic acid) grafting with polyethylene glycol-CA4 bonded pharmaceutical compound

585mg of poly(glutamic acid) grafted with polyethylene glycol prepared in Example 4 together with 316mg, 252.8 mg, or 190mg of CA4 were added to a dry reaction bottle respectively, and dissolved with 20ml of dry N,N-dimethylformamide. 25mg of 4-dimethylaminopyridine and 252.4 mg of N,N'-diisopropyl carbodiimide were then added, and the reaction was performed with stirring at room temperature under a nitrogen atmosphere for 48h. It was taken in ethyl ether, and filtered. The solid was collected and vacuum-dried at room temperature. The solid was re-dissolved with N,N-dimethylformamide, and freeze-dried to give poly(glutamic acid) grafting with polyethylene glycol-CA4 bonded drug. The CA4 content and yield could be seen in Table 2. Table 2 showed the preparation method yield of bonded pharmaceutical compounds provided in Examples 12-14 of this invention and the CA4 content obtained in the bonded pharmaceutical compounds.

**Table 2**

| Example | CA4 content (%) | Yield (%) |
|---|---|---|
| 12 | 35.0 | 82.2 |
| 13 | 28.1 | 85.5 |
| 14 | 20.7 | 80.8 |

### Examples 15-17

### Preparation of poly(aspartic acid) grafting with polyethylene glycol-CA4 bonded pharmaceutical compound

552mg of poly(aspartic acid) grafted with polyethylene glycol prepared in Example 7 together with 316mg, 252.8 mg, or 190mg of CA4 were added to a dry reaction bottle respectively, and dissolved with 20ml of dry N,N-dimethylformamide. 25mg of 4-dimethylaminopyridine and 252.4 mg of dicyclohexyl carbodiimide were then added, and the reaction was performed with stirring at room temperature under a nitrogen atmosphere for 48h. Filtration was performed, and it was taken in ethyl ether. The solid was collected and vacuum-dried at room temperature. The solid was re-dissolved with N,N-dimethylformamide, and freeze-dried to give a poly(aspartic acid) grafting with polyethylene glycol-CA4 bonded drug. The CA4 content and yield could be seen in Table 3. Table 3 showed the preparation method yield of bonded pharmaceutical compounds provided in Examples 15-17 of this invention and the CA4 content obtained in the bonded pharmaceutical compounds.

**Table 3**

| Example | CA4 content (%) | Yield (%) |
|---|---|---|
| 8 | 34.0 | 81.2 |
| 9 | 27.2 | 81.5 |
| 10 | 20.0 | 81.8 |

### Examples 18-20

### Preparation of poly(aspartic acid) grafting with polyethylene glycol-CA4 bonded pharmaceutical compound

552mg of poly(aspartic acid) grafted with polyethylene glycol prepared in Example 8 was added to each of dry reaction bottles respectively, and dissolved with 20ml of dry N,N-dimethylformamide. Thereafter, 0.191 mL of dry triethylamine and 0.215 mL of 2,4,6-trichlorobenzoyl chloride were added. After placing in an oil bath at 60°C, stirring was performed for 10min. Thereafter, 316mg, 253mg, or 190mg of CA4 and 135mg of 4-dimethylaminopyridine were added respectively under a nitrogen atmosphere for continuing the reaction at room temperature for 12h. After completion of the reaction, the reaction solution was taken in ethyl ether, and filtered. The solid was collected and vacuum-dried at room temperature. The solid was re-dissolved with DMF, and freeze-dried to give a poly(glutamic acid) grafting with polyethylene glycol-CA4 bonded pharmaceutical compound. The weight was measured, and the yield was calculated. The CA4 content and yield could be seen in Table 4. Table 4 showed the preparation method yield of bonded pharmaceutical compounds provided in Examples 18-20 of this invention and the CA4 content obtained in the bonded pharmaceutical compounds.

**Table 4**

| Example | CA4 content (%) | Yield (%) |
|---|---|---|
| 18 | 32.0 | 85.2 |
| 19 | 26.2 | 80.5 |
| 20 | 22.0 | 84.8 |

### Example 21

### Characterization of a polymer-bonded CA4 pharmaceutical compound

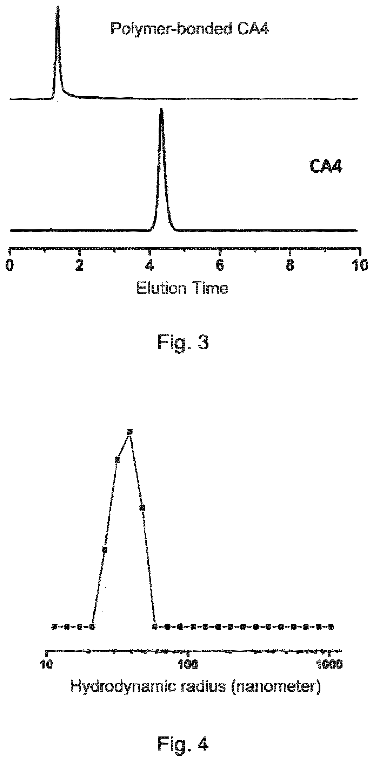

It was determined by HPLC analysis that no unbonded CA4 was present in the product. In the HPLC, the mobile phase was acetonitrile/water = 4/1, and the peak appearance time of the small molecule CA4 was 3.5 minutes. The results could be seen in Fig. 3. Fig. 3 showed HPLC graphs of a small molecule CA4 and the polymer-bonded CA4 pharmaceutical compound prepared in Example 10. As could be seen from this figure, no free CA4 was present in the polymer-bonded CA4 pharmaceutical compound prepared in Example 10.

The resultant polymer-bonded CA4 pharmaceutical compound was subjected to dynamic light scattering analysis to measure the hydrodynamic radius of micelles formed by self-assembling. Fig. 4 showed the dynamic light scattering result of the polymer-bonded CA4 pharmaceutical compound prepared in Example 10 at a concentration of 0.2 mg/mL in water. As could be seen from this figure, the hydrodynamic radius of self-assembled micelles was between 20-60nm, and the particle size distribution was uniform.

### Example 22

### In vitro simulated release of polymer-bonded CA4 pharmaceutical compound

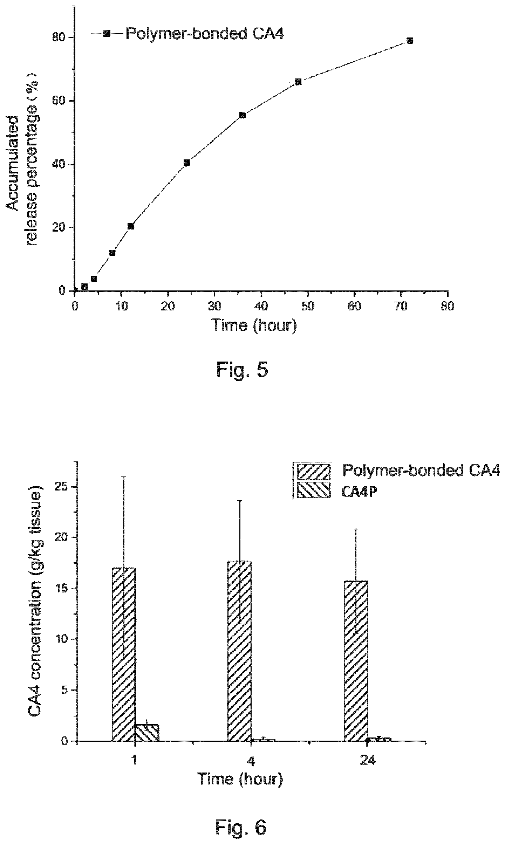

3mg of the polymer-bonded CA4 drug prepared in Example 10 was accurately weighed, dissolved in 5mL of phosphate buffer (pH 7.4), loaded to a dialysis bag and then placed in 45mL of phosphate release solution, and shaken in a homeothermic oscillating tank at 37°C. 3mL of the released solution was taken out at time point of 2, 4, 8, 12, 24, 36, 48, and 72 hours respectively, and the CA4 content was measured by ultraviolet. Finally, the accumulated release amount of CA4 within 72 hours was calculated, and the results could be seen in Fig. 5. Fig. 5 showed the release result of the bonded pharmaceutical compound prepared in Example 10 in a simulated body fluid. As could be seen from Fig. 5, the bonded pharmaceutical compound of CA4 slowly released CA4 in a simulated body fluid, and there was no phenomenon of burst release.

### Example 23

### Tumor distribution of a polymer-bonded CA4 pharmaceutical compound compared to CA4P

24 Balb/C mice (5-6 weeks old, female, body weight of approximately 20g) were utilized, and C26 murine colon cancer cells were seeded at the right underarm at 2.0 × 10⁶/mouse, respectively. When tumor volume was grown to about 200mm³, the mice were divided into 2 groups, which were administered with CA4P and the polymer-bonded CA4 pharmaceutical compound prepared in Example 10 via tail vein injection, respectively. The administration dosage was 4.0 mg CA4/kg body weight. The mice were sacrificed after 1, 4, and 24 hours. Tumor was collected and homogenized. The CA4 concentration was measured by HPLC. Drug/kg weight in tumor of the 2 groups of samples obtained was shown in Fig. 6. Fig. 6 showed CA4 drug concentrations in tumor tissues after administration of a polymer-bonded CA4 pharmaceutical compound and CA4P in Example 23. As could be seen from this figure, there was a significant difference in intra-tumor CA4 drug retention and enrichment between the CA4 drug and the small molecule CA4P. The polymer-bonded CA4 drug could maintain the CA4 drug content in tumor for a very long period of time, and thus was capable of exerting a continuous inhibition effect on the growth of tumor. This result showed the superiority of this polymer-bonded CA4 drug in the treatment of tumor.

### Example 24

### Therapeutic effect on tumor of a polymer-bonded CA4 pharmaceutical compound by single administration

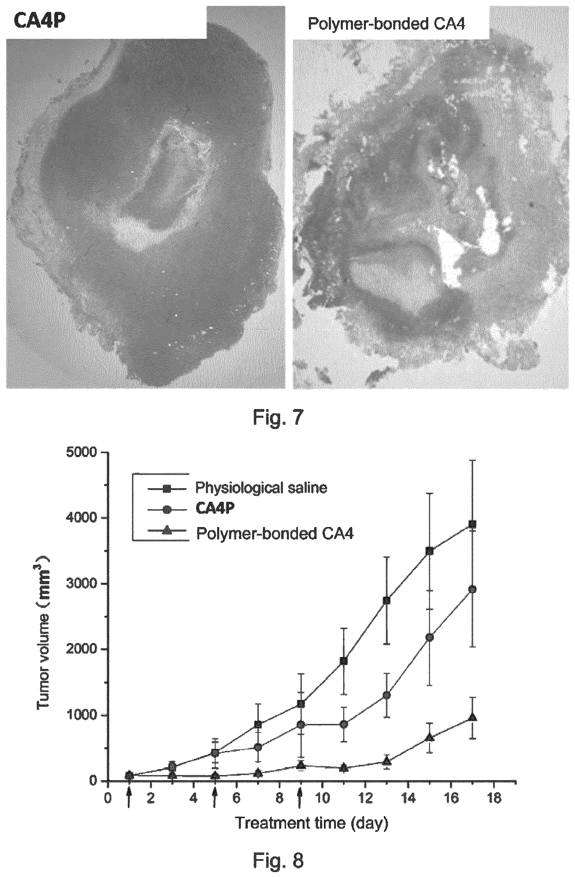

6 Balb/C mice (5-6 weeks old, female, body weight of approximately 20g) were utilized, and C26 murine colon cancer cells were seeded at the right underarm at 2.0× 10⁶/mouse, respectively. When tumor volume was grown to about 200mm³, the mice were divided into 2 groups, which were administered with CA4P and the polymer-bonded CA4 drug prepared in Example 10 via tail vein injection, respectively. The administration dosage was 50mg CA4/kg body weight. The mice were sacrificed after 72 hours. Tumor was collected, and pathological H&E analysis was performed. The result was shown in Fig. 7. Fig. 7 showed the pathological analysis of tumor after single administration of CA4P and the polymer-bonded CA4 drug measured in Example 24. As could be seen from this figure, after treatment by administration for 72 hours, a wide range of recurrence occurred in the tumor of the CA4P treatment group, while the polymer-bonded CA4 drug could continuously inhibit the growth of tumor. This well demonstrated the therapeutic advantages of the polymer-bonded CA4 drug designed by this invention.

### Example 25

Tumor inhibition effect of polymer-bonded CA4 pharmaceutical compound 18 Balb/C mice (5-6 weeks old, body weight of about 20g) were utilized, and 2.0× 10⁶ C26 cells were seeded at the right underarm, respectively. When tumor was grown to 100mm³, the mice were equally divided into 3 groups (a physiological saline group, a CA4P group, and a group of the polymer-bonded CA4 drug prepared in Example 10), and this was recorded as day 0. Thereafter, administration was performed 3 times on day 1, 5, and 9, respectively. The administration dosage was 50.0 mg CA4/kg body weight. Tumor was measured 3 times per week and body weights of mice were recorded, and observation was not stopped until day 17. Diagrams of the tumor volume were shown in Fig. 8, respectively. Fig. 8 showed the therapeutic effects of a polymer-bonded CA4 pharmaceutical compound and CA4P on tumor measured in Example 25. As could be seen from the results, an excellent tumor inhibition rate of 73.6% was obtained in the polymer-bonded CA4 pharmaceutical compound group, while a tumor inhibition rate of 24.0% was obtained in the CA4P group. The results indicated that the polymer-bonded CA4 drug provided by this invention was safe and effective, had a therapeutic effect superior to that of a CA4P at the same dosage, and had great potentiality in the treatment of solid tumors.

The description of the above Examples is only used to help the understanding of the method of this invention and the core idea thereof. It shall be indicated that, for the person skilled in the art, various improvements and modifications may also be made to this invention without departing from the principle of this invention. These improvements and modifications also fall in the protection scope of the claims of this invention.

## Claims

1. A polymer-bonded CA4 pharmaceutical compound having a structure represented by formula (I), 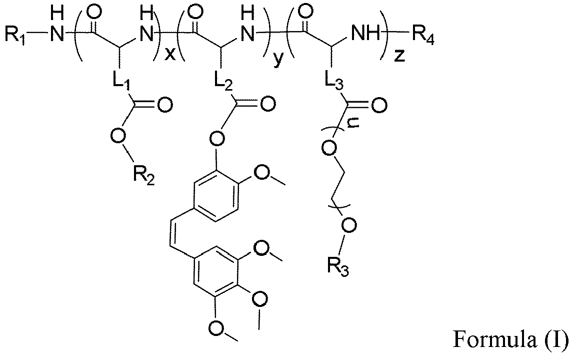 wherein,
R₁ is selected from a C2-C10 linear alkyl group, a C3-C10 branched alkyl group, or a C6-C20 aryl group;
R₂ is selected from a hydrogen atom or a cation;
R₃ is selected from an unsubstituted C1-C20 alkyl group or a substituted C1-C20 alkyl group;
R₄ is selected from a hydrogen atom or a C1-C6 alkyloyl group;
L₁, L₂, and L₃ are independently selected from -CH₂- or -CH₂CH₂-;
x, y, and z represent polymerization degree, and 10 ≤ x+y+z ≤ 5000, wherein x ≥ 0, y > 0, z > 0; and
n represents polymerization degree, and 10 ≤ n ≤ 500.

2. The bonded pharmaceutical compound according to claim 1, wherein said R₁ is a C3-C8 linear alkyl group, a C5-C8 branched alkyl group, or a C8-C15 aryl group.

3. The bonded pharmaceutical compound according to claim 1, wherein said R₂ is selected from a hydrogen atom, a metal cation, or an organic cation.

4. The bonded pharmaceutical compound according to claim 1, wherein said R₂ is selected from a hydrogen atom, a sodium ion, a potassium ion, an ammonium ion, or a positively charged amino acid ion.

5. The bonded pharmaceutical compound according to claim 1, wherein said R₃ is selected from an unsubstituted C2-C20 linear alkyl group, an unsubstituted C3-C20 branched alkyl group, a substituted C2-C20 linear alkyl group, or a substituted C3-C20 branched alkyl group.

6. The bonded pharmaceutical compound according to claim 5, wherein the substituent in said substituted C2-C20 linear alkyl group is one or more of a hydroxy group, an aldehyde group, an amino group, a mercapto group, and a saccharide residue; and
the substituent in said substituted C3-C20 branched alkyl group is one or more of a hydroxy group, an aldehyde group, an amino group, a mercapto group, and a saccharide residue.

7. The bonded pharmaceutical compound according to claim 1, wherein said R₄ is selected from a hydrogen atom, an acetyl group, or a propionyl group.

8. The bonded pharmaceutical compound according to claim 1, wherein a value range of said x, y, and z is 30 ≤ x+y+z ≤ 300.

9. A preparation method of a polymer-bonded CA4 pharmaceutical compound, comprising:
reacting a copolymer compound having a structure of formula (II) with CA4 in the presence of a condensation agent, 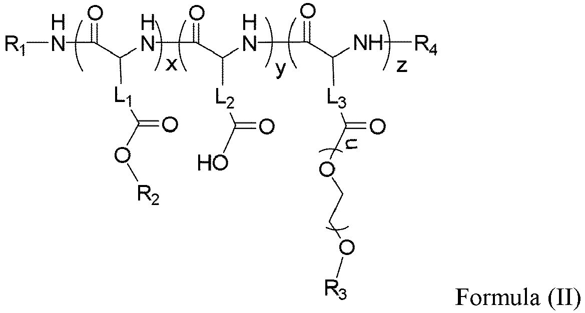 to give a polymer-bonded CA4 pharmaceutical compound having a structure of formula (I): 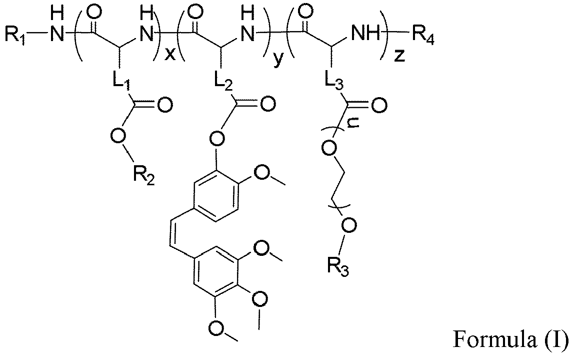 wherein,
R₁ is selected from a C2-C10 linear alkyl group, a C3-C10 branched alkyl group, or a C6-C20 aryl group;
R₂ is selected from a hydrogen atom or a cation;
R₃ is selected from an unsubstituted C1-C20 alkyl group or a substituted C1-C20 alkyl group;
R₄ is selected from a hydrogen atom or a C1-C6 alkyloyl group;
L₁, L₂, and L₃ are independently selected from -CH₂- or -CH₂CH₂-;
x, y, and z represent polymerization degree, and 10 ≤ x+y+z ≤ 5000, wherein x ≥ 0, y > 0, z > 0; and
n represents polymerization degree, and 10 ≤ n ≤ 500.

10. The preparation method according to claim 9, wherein said condensation agent is 2,4,6-trichlorobenzoyl chloride, N,N-diisopropyl carbodiimide, or dicyclohexyl carbodiimide.

11. Use of the bonded pharmaceutical compound according to any one of claims 1-8 in the preparation of a drug for treating tumors.
